# EUROPEAN PATENT APPLICATION

(11) **EP 4 792 785 A1**
(43) Date of publication of application: **19.08.2026**
(21) Application number: 25158046.0
(22) Date of filing: 14.02.2025
(51) Int. Cl.: A61F 4/00, A61F 2/78

(54) **DEVICE FOR ESTABLISHING A MECHANICAL CONNECTION BETWEEN A LIMB OF A PERSON AND A TOOL, AND ORTHOTIC SYSTEM WITH SUCH A DEVICE**

(71) Applicant: macu4 AG, 8001 Zürich (CH)
(72) Inventor: Chevrot, Alec, 2505 Biel (CH); Schiller, Lukas, 8006 Zurich (CH)
(74) Representative: Kreuels, Justus

(57) **Abstract**

The present invention relates to a device (100) for establishing a mechanical connection between a limb of a person and a tool (300), such that a load transfer is enabled from the limb to the tool and vice versa, the device comprising a limb connection system (210) configured to achieve a form-fitting and/or force-fitting attachment to the limb, further comprising a holding mount (220) in which the tool can be positioned, wherein the holding mount comprises at least three bearing portions (221, 222, 223), which are configured to collectively bear the tool, wherein a first bearing portion (221) is located at a first holding member (230) protruding from a main body (240) of the holding mount, wherein the first holding member and the main body together form a first receptacle (280) for a first section (310) of the tool (300), and wherein the first holding member has elastic properties such that it is deformable in relation to the main body for allowing placement of the first section in the first receptacle.

Further, the present invention relates to an orthotic system, comprising the device and a cuff (1).

## Description

The present invention relates to a device for establishing a mechanical connection between a limb of a person and a tool as well as to an orthotic system with such a device.

In the case of a congenital deformity or an amputation, or neurological or musculoskeletal issues, such as hemiplegia and rheumatism, it may be difficult to perform bimanual activities, such as eating and playing drums. Moreover, it may be difficult to perform activities which are desired to be performed with a preferred hand, such as writing. Daily life for affected people is full of little challenges that an ordinary person would never suspect, let alone imagine. People with a birth defect learn to live with it, but outside help is welcome to ease the effort and mental burden. For people who have suffered an amputation as a result of an accident, for example, it is necessary to relearn skills. Tools can facilitate not only their daily life but also their rehabilitation. Similarly, in the event of an accident, surgery or injury, hands may become weakened, sensitive, immobilized for a limited period. Therefore, a rapid provision of support is essential to enable affected individuals to continue their activities and daily routines.

An orthotic system, also called ortho-prosthetic system, can be designed for individuals with partial or complete loss of hand function. This loss may be due to temporary or permanent weakening, hand injury, paralysis, neuromuscular disease, partial absence of the hand or absence due to wrist disarticulation. Such an orthotic system may be worn on the forearm of a person and is intended to assist individuals with a disability below the wrist. This encompasses various scenarios, including those with only a portion of the palm remaining, small residual fingers, a limited number of fingers, or limited and painful movement capacity. In consequence, the orthotic system should be engineered to hold cutlery, a drumstick, a pen/pencils or similar structures and to transmit a tensile or compressive load from the object to the forearm. The orthotic system may comprise or may itself form a device for stablishing a mechanical connection between a limb of a person and a tool.

Existing solutions are often poorly adapted, expensive and restricting in terms of the activities they support. Moreover, existing solutions often require a skilled person to use the orthotic system or device. Also, some existing solutions have hinged or similarly moved parts used for grabbing a tool and are thus not only expensive in manufacturing, but also prone to malfunction, so that short maintenance intervals are required.

The document CN 213 665 988 U discloses a device for holding a violin bow. Due to lack of flexibility, the device can only be used for the violin bow and no other tool.

The technical objective of the present invention is to provide a device and an orthotic system, which overcome the before-stated disadvantages and ensure effective load transfer from and/or to a tool, while allowing simple and relatively quick application to a limb.

This technical objective is solved by the subject-matter of the independent claims. Preferred embodiments of the invention are defined in dependent claims and the description. It should be noted that a skilled person will combine the individual features in a technologically sensible way and thus will arrive at further embodiments in the scope of the invention.

A first aspect of the invention relates to a device for establishing a mechanical connection between a limb of a person and a tool, such that a load transfer is enabled from the limb to the tool and vice versa, the device comprising a limb connection system configured to achieve a form-fitting and/or force-fitting attachment to the limb, further comprising a holding mount in which the tool can be positioned, wherein the holding mount comprises at least three bearing portions, which are configured to collectively bear the tool, wherein a first bearing portion is located at a first holding member protruding from a main body of the holding mount, wherein the first holding member and the main body together form a first receptacle for a first section of the tool, and wherein the first holding member has elastic properties such that it is deformable in relation to the main body for allowing placement of the first section in the first receptacle.

By connecting the holding mount to the tool, the device works as a support for a person with a congenital deformity or an amputation or neurological or musculoskeletal issues, such as hemiplegia and rheumatism. The load that can be transferred or transmitted or applied from the limb to the tool or from the tool to the limb may be a tensile or compressive load. The load may result in or from external forces acting on the tool.

The holding mount is configured to passively bear the tool, i.e. no force needs to be (actively) applied to hold the tool, as it is, for instance, necessary at devices with hinged components. The holding mount is configured to fix the tool in at least one translational degree of freedom, which can mean securing the tool or limiting its movement in at least one translational degree of freedom. The holding mount is preferably configured to fix the tool in at least two translational degrees of freedom. The holding mount may be configured to fix the tool in at least one rotational degree of freedom. The holding mount preferably has only three bearing portions.

A connection between the tool and the holding mount can be form-fitting and/or force-fitting. The connection between the tool and the holding mount preferably is form-fitting and force-fitting. While a form-fitting connection may be achieved by a form of the holding mount, for instance, of the first receptacle, a force-fitting connection may be achieved by the elastic properties or flexibility of the first holding member (e.g. resilience) or by the material used to produce the holding mount or parts of it (e.g. friction force). As the limb connection system allows a form-fitting and/or force-fitting attachment of the device to the limb, and the holding mount allows a form-fitting and/or force-fitting connection between the device and the tool, and the limb connection system is mechanically connected to the holding mount, the tool can be connected to the limb via the device by means of a form-fitting and/or force-fitting connection.

The first bearing portion may bear the first section of the tool at a contact point, contact line or a contact area. The first bearing portion preferably bears the first section of the tool at a contact area, so that a first bearing force acts on the contact area located in the first section of the tool. A first bearing pressure may result. The first bearing force or the first bearing pressure, respectively, act in a first force direction.

The first receptacle may receive the first section of the tool, such that the first section or a cross-section of the tool at the first section is completely inside the first receptacle. It is possible that the tool or first section of the tool also touches other parts of the first holding member or the main body, apart from the first bearing portion. The tool or first section of the tool may even be borne by other parts of the first holding member or the main body. The main body may have massive appearance, such that it differs from a bar-like structure.

The elastic properties of the first holding member may provide flexibility for the insertion of the tool into the first receptacle. In particular, the first holding member may be bendable, such that a distal end the first holding member can be moved with respect to the main body. The elastic properties of the first holding member may provide applicability of force to the tool and/or damping of vibrations (e.g. when the tool is a drumstick).

The tool may be of at least two different types. The tool may be a knife, fork, spoon or any other type of cutlery, or a pencil or pen or any other type of writing utensil, or drumsticks, wherein this list is not exhaustive. The first section of the tool may be a first portion or first part of the tool, in case the tool has different portions or parts. The tool may be exposable to external forces and/or may be used to apply external forces. For instance, the knife can be used to cut bread or a steak. In such a case, the load can be transferred by means of the limb connection system from the limb of the person to the device, then from the limb connection system to the holding mount, and then by means of the holding mount from the device to the knife, wherein the knife can apply external forces that are necessary for cutting to the bread or steak.

The limb connection system is preferably configured to be attached to a forearm of a person. The limb connection system may be or may comprise an adapter for a limb accessory or a part thereof, wherein the limb accessory may be in the form of a cuff (see description below). Alternatively, the limb connection system may be or may comprise a socket wearable at the limb or a part thereof. The load can then be transferred via the adapter and the limb accessory or via the socket from the limb to the holding mount or vice versa.

When being or comprising an adapter, the limb connection system may comprise an insertion element configured to be pushed into a retainer and/or a first part of a ratchet system, wherein the first part of the ratchet system may be an integral part of or arranged at the insertion element. Alternatively, the limb connection system may comprise the retainer.

The insertion element may be designed to be pushed along a slide-in direction into the retainer at the limb accessory. The insertion element may be configured to be repeatedly attached to and removed from the retainer. The insertion element may be configured to be interlocked with the retainer in order to mechanically secure the device to the limb accessory. The insertion element may have a transversal extension and a longitudinal extension longer than the transversal extension, wherein the insertion element can be inserted along its longitudinal extension into the retainer, such that at least one translational degree of freedom perpendicular to the slide-in direction of the insertion element is blocked or can be blocked by the retainer.

The first part of a ratchet system may have at least one form element, which is capable of establishing a positive locking with a designated counterpart (second part) of the ratchet system located at the limb accessory. The form element may have the shape of a tooth or recess. Other shapes of the form element are possible, such as a partial circle. The ratchet system particularly provides a latching function, so that the ratchet system may be used to mechanically connect and secure the device to the limb. The first part of a ratchet system may have one or more rows of form elements, in particular teeth or recesses, and the designated counterpart may have one or more rows of form elements, which are complementary in form and size.

The device according to the invention has the advantage that a tool can be applied to it in a simple and relatively quick way. Furthermore, the device according to the invention ensures effective load transfer from and/or to a tool, such that many activities are supported. Also, the device according to the invention can be manufactured in a relatively cost-efficient way. The device according to the invention as well allows omission of hinged parts, so that relatively long maintenance intervals may be achieved. Moreover, with the device according to the invention different types of tools can be used.

In a preferred embodiment, the distal end of the first holding member is arranged adjacent to the main body.

Adjacent means that the distal end of the first holding member is preferably arranged next to and spaced apart from the main body, so there is a gap between the distal end of the first holding member and the main body. The first holding member and the main body preferably do not touch each other. The first receptacle may have a slot-like shape. Such an arrangement allows a smooth placement of the tool in the first receptacle, as the tool may be used to deform, in particular bend, the first holding member.

In a preferred embodiment of the device, a second bearing portion is located at a second holding member protruding from the main body, wherein the second holding member and the main body together form a second receptacle for a second section of the tool.

The second bearing portion may bear the second section of the tool at a contact point, contact line or contact area. The second bearing portion preferably bears the second section of the tool at a contact area, so that a second bearing force acts on the contact area located in the second section of the tool. A second bearing pressure may result. The second bearing force or the second bearing pressure, respectively, act in a second force direction, which preferably is substantially parallel to the first force direction and may act directionally equal. The second receptacle may receive the second section of the tool, such that the second section or a cross-section of the tool at the second section is completely inside the second receptacle. It is possible that the tool or second section of the tool also touches other parts of the second holding member or the main body, apart from the second bearing portion. The tool or second section of the tool may even be borne by other parts of the second holding member or the main body.

In a preferred embodiment, the first holding member and/or the second holding member has a bridge-shaped and/or arc-shaped and/or J-shaped and/or S-shaped cross-section.

In other words, the first holding member may protrude from the main body in a bridge-shaped and/or arc-shaped and/or J-shaped and/or S-shaped manner. Similarly, the second holding member may protrude from the main body in a bridge-shaped and/or arc-shaped and/or J-shaped and/or S-shaped manner. The terms "L-shaped" or "half-U-shaped" can be synonymously used for the term "J-shaped". The first holding member or second holding member having a J-shaped cross-section may result in the first receptacle or second receptacle, respectively, having an essentially U-shaped cross-section. The first holding member or second holding member having a S-shaped cross-section may result in the first receptacle or second receptacle, respectively, having an essentially B-shaped cross-section. The first holding member and/or second holding member may extend perpendicular to the bridge-shape or arc-shape or J-shape or S-Shape, so that the first bearing portion and/or second bearing portion can be areal. The bridge-shaped and/or arc-shaped and/or J-shaped and/or S-shaped cross-section may be in different planes. The planes may be arranged essentially orthogonal to each other. For instance, the second holding member may have a bridge-shaped cross-section in one plane and an arc-shaped cross-section in another plane, so that the second holding member may have a bridge-like appearance when the holding mount is oriented in one orientation and may have an arc-like appearance when the holding mount is oriented in another differing orientation.

In a preferred embodiment, the second holding member has elastic properties such that it is deformable in relation to the main body for allowing placement of the second section in the second receptacle and/or for allowing placement of the first section in the first receptacle.

The elastic properties of the second holding member, optional in combination with its design, can contribute to a force-fitting connection between the tool and the holding mount. The elastic properties of the second holding member may provide flexibility for insertion of the tool into the first receptacle and/or the second receptacle. The elastic properties of the second holding member may provide applicability of force to the tool and/or damping of vibrations (e.g. when the tool is a drumstick). When positioning the tool in the holding mount, the tool may be placed in the second receptacle (not necessarily with its second section) and then the second holding member may be deformed, preferably bent, in order to place the tool, preferably the first section of the tool, in the first receptacle. A further step may be foreseen to push the tool, preferably in a longitudinal direction, such that the first section of the tool is placed in the first receptacle and the second section of the tool is placed in the second receptacle. The elastic properties of the first holding member may similarly be such that they allow placement of the second section of the tool in the second receptacle.

In a preferred embodiment, a third bearing portion is located at the main body.

The described first bearing portion, second bearing portion and third bearing portion preferably are the three bearing portions that collectively bear the tool. The holding mount is preferably able to bear the tool solely via the first, second and third bearing portions. The third bearing portion may bear a third section of the tool. The third bearing portion is preferably arranged such that it is located at an opposite site of the tool (arranged in the holding mount) than the first bearing portion and the second bearing portion. The third bearing portion is preferably arranged such that the third section of the tool (arranged in the holding mount) is between the first section and the second section on a longitudinal extension of the tool. The third bearing portion may bear the third section of the tool at a contact point, contact line or contact area. The third bearing portion preferably bears the third section of the tool at a contact area, so that a third bearing force acts on the contact area located in the third section of the tool. A third bearing pressure may result. The third bearing force or the third bearing pressure, respectively, act in a third force direction, which preferably is substantially parallel to the first force direction and second force direction. However, the third bearing force or third bearing pressure acts in a substantially opposite direction to the first bearing force or first bearing pressure and the second bearing force or second bearing pressure. The main body may have elastic properties at the third bearing portion. The third bearing portion preferably provides high friction resistance, which may result from its shape and/or material.

In a preferred embodiment, the second holding member protrudes from the main body in a different plane than the first holding member.

The first holding member may protrude from the main body in a first plane. The second holding member may protrude from the main body in a second plane. The first plane and the second plane may differ from each other, such that an angle between the first plane and second plane may amount to 45 to 140 degrees, preferably 80 to 110 degrees, more preferably to 90 to 100 degrees. It has been found out that the usability of the device is best with an angle between the first plane and second plane in the range of 80 to 110 degrees.

In a further preferred embodiment, at least one of the first holding member and second holding member comprises at least one nose and/or lip and/or tooth, which at least supports to bear the tool such that the tool is exposable to external forces in at least two directions, which may be essentially oriented orthogonally to each other.

The first holding member may have a nose, e.g. for holding the tool in place at the first bearing portion. In particular, such a nose may be foreseen in case the first holding member has a J-shaped cross-section. The nose preferably is located at the distal end of the first holding member. A shape of the nose may be such that it allows smooth insertion of the tool into the first receptacle. With the nose the first holding member may have a hook-like or hook-shaped cross-section. The nose may be particularly advantageous in cases, where an external force is applied to the tool with at least one force component that affects a movement of the tool at the first section, which is directed towards the gap between the first holding member and the main body. The nose may be omitted in case the first holding member has a S-shaped cross-section. The second holding member may comprise at least one tooth, preferably two teeth. The teeth may extend substantially orthogonally to the bridge-shaped cross-section. The two teeth preferably extend in the same direction. The two teeth are preferably arranged with a distance to each other, so that the second section of the tool may be placed between them. With such teeth the second holding member may have an appearance of an upper jaw of a snake.

In a preferred embodiment, at least one of the at least three bearing portions has a groove, which enables fixation of a tool with a flat shape.

The tool may have a flat shape in the first section and/or second section and/or third section. Such a flat shape is, for instance, typical for cutlery made from steel. Preferably, the first bearing portion and the second bearing portion and the third bearing portion have at least one groove. In case the first holding member has a nose, the at least one groove is located underneath the nose. In case the second holding member has two teeth, the at least one groove is located between the two teeth. Preferably, more than one groove is foreseen, so that the tool with the flat shape can be locked in different positions or orientations. The tool may be snaped from one groove to the other, in order to change its orientation, wherein the change of orientation may be a rotation about the tool's longitudinal axis. When more than one grove is foreseen, preferably, the same number of grooves is foreseen at each of the at least three bearing portions.

In a preferred embodiment, the device comprises a ball joint, by means of which the holding mount is rotatable in relation to the limb connection system.

In other words, the holding mount may be rotatably connected to the limb connection system. Alternatively, the holding mount may be fixedly connected to the limb connection system. By means of the ball joint the holding mount can be rotatable relative to the limb of the person. This allows adjustability of an angle between the holding mount and the limb, so that user comfort is improved and the device's versatility in connecting to various kinds of tools is expanded. The holding mount may be rotatable in relation to the limb connection system around at least one axis, so that a pivoting movement of the holding mount in at least one rotational degree of freedom is enabled. The rotating or pivoting movement can be enabled in different rotational degrees of freedom. The rotating or pivoting movement may be a 360 degree movement. The rotating or pivoting movement may be limited across different rotational degrees of freedom, for instance, to around 90 degrees.

The ball joint may be arranged at a distal end of the limb connection system, in particular at a distal end of the insertion element. The ball joint may be part of the limb connection system and/or the holding mount. A ball of the ball joint may be connected to or an integral part of the limb connection system, preferably the insertion element. Alternatively, the ball may be connected to or an integral part of the holding mount, preferably the main body. A counterpart to the ball, which is at least partly covering the ball, may be connected to or an integral part of the holding mount, preferably the main body. Alternatively, the counterpart to the ball may be connected to or an integral part of the limb connection system, preferably the insertion element.

A locking mechanism may be foreseen, which serves for locking the ball joint, such that an orientation of the holding mount in relation to the limb connection system can be locked. The locking mechanism may be configured to brace the counterpart around the ball in order to cause friction between the ball and the counterpart. Such a locking mechanism may comprise a lever to loosen or tighten the locking mechanism. When the locking mechanism is in an unlocked status, the holding mount may be rotatable in relation to the limb connection system. When the locking mechanism is in a locked status, a position or orientation of the holding mount in relation to the limb connection system is locked.

In a preferred embodiment, the holding mount is made one-piece and/or the holding mount or parts of the holding mount are made from a rubber-like material.

The holding mount is preferably produced using additive manufacturing, more preferably 3D printing, more preferably the technique of Multi Jet Fusion (MJF) or Selective Laser Sintering (SLS) or Fused Deposition Modeling (FDM), also called Fused Filament Fabrication (FFF), or Stereolithography (SLA) or Digital Light Processing (DLP) or Direct Ink Writing (DIW) or Liquid Deposition Modeling (LDM). The holding mount or parts of the holding mount may alternatively be produced using injection moulding.

The holding mount may be produced as a single piece. Instead of one-piece, the holding mount may comprise more than one piece or part, wherein the different pieces or parts may be made from different materials. The term "made from" does also include that the holding mount or parts of it are covered by the respective material.

The rubber-like material has the advantage that the tool is nicely held in place due to high friction resistance. The rubber-like material may be thermoplastic elastomer and/or silicone rubber and/or polyurethane rubber, such as thermoplastic polyurethane (TPU), and/or flexible resins, while this list is not exhaustive. Alternatively, other polymers, such as polyamide 12 (PA12), may be used for the holding mount or parts of it. Preferably, at least one of the bearing portions, in particular the third bearing portion, is made from the rubber-like material, in particular TPU. Further preferably, at least the first holding member and/or the second holding member are made from the rubber-like material, in particular TPU. The material of the (remaining) main body may be made from a different material, such as PA12 or similar. More preferably, the whole holding mount is made from rubber-like material, in particular TPU. In case the limb connection system comprises the insertion element, the insertion element preferably is made from PA12. In case the device comprises a ball joint, the ball or the counterpart to the ball is preferably made from rubber-like material like TPU, in order to ensure a proper function of the ball-joint. Depending on the material used for producing the limb connection system, in particular the insertion element, and the holding mount, the ball and/or counterpart may be produced at least partially separately from remaining parts.

A further aspect of the invention relates to an orthotic system comprising the described device and a cuff mechanically connectable or connected to the device by means of the limb connection system.

It should be noted that the design features and advantages described in connection with the device are also applicable and transferable to the orthotic system and vice versa.

The term "ortho-prosthetic" may be used synonymously to the term "orthotic", so that by claiming the orthotic system an ortho-prosthetic system is claimed.

The orthotic system comprising the device and the cuff wearable at the limb of the person can be a convenient system for establishing a connection between the limb and the tool. Such an orthotic system comprising the cuff is to be understood as a preferred embodiment. However, other embodiments of the orthotic system are possible, for instance, using a socket or any other structure that is attachable to the limb. That means, as an alternative to the cuff, a socket may be used, which may represent the limb connection system or is part thereof.

The cuff can serve as a pleasant limb accessory for creating a reversible connection to the limb, in particular the forearm of a person. The person can connect the device to the cuff and consequently benefits from all the functions of the device, enabling performing tasks that typically require the use of hands. The person can, for instance, use cutlery or a pen/pencil or a drumstick by connecting the (handle of the) cutlery or pen/pencil or drumstick to the holding mount.

The cuff mechanically connected to the device includes embodiments, where the device is integrally formed with the cuff.

In a preferred embodiment, the cuff comprises a cuff base and a fixation means, wherein the cuff base and the fixation means can be combined or are combined via direct or indirect interlocking with each other into a sleeve attachable to the limb, wherein the sleeve can be tightened around the limb by reducing a circumference formed by the cuff base and the fixation means, such that the form-fitting and/or force-fitting attachment to the limb is achieved.

The form-fitting and/or force-fitting attachment to the limb allows the load transfer from the cuff to the limb and vice versa. The cuff base may be in the form of a lower shell, wherein the lower shell and the fixation means provide a system that can be tightened around the limb by reducing the circumference formed by the lower shell and the fixation means. The fixation means may be a cuff top in form of an upper shell and/or a strap system and/or a lacing system. Such a cuff design with its division into the cuff base and the fixation means facilitates the insertion of the limb into the cuff, which can be difficult due to an enlargement at the beginning of the palm. Further, such a cuff design provides a fast and comfortable way to interlock the cuff with the forearm. The possible reduction of the circumference can be achieved by using the strap system and/or the lacing system. The lacing system may have a lacing element and a lacing knob, wherein the lacing element is connected to the lacing knob. Tension in the lacing element may be generated by turning the lacing knob.

In a preferred embodiment of the orthotic system, the device comprises a first part of a ratchet system and the cuff comprises a second part of the ratchet system, wherein the first part and the second part each have at least one form element, which are complementary in form and size, in order to establish a positive locking.

The ratchet system particularly provides a latching function for connecting the device to the cuff or vice versa. By interlocking the first part with the second part the device can be mechanically connected to the cuff. The at least one form element, may have the shape of a tooth or recess. Other shapes of the form element are possible, such as a partial circle. The first part may have multiple form elements, in particular teeth or recesses, and/or the second part may have multiple form elements, which are complementary in form and size. Preferably, a plurality of form elements of the first part and a plurality of form elements of the second part are arranged in rows, respectively, so that the positive locking can be realized at different positions of the device in relation to the cuff. A plurality of teeth may form a serration. The arrangement in rows can allow for incremental movement in at least one direction and thus adjustability of the fixture point of the device at the cuff. The adjustment of the fixture point can, for example, be executed by disengaging, repositioning, and relocking the ratchet system.

In a further preferred embodiment of the orthotic system, the cuff or the device comprises a retainer and the respective other one of the cuff and the device comprises an insertion element, wherein the insertion element is configured to be pushed into the retainer along a slide-in direction.

Preferably, the cuff comprises the retainer and the device comprises the insertion element. The insertion element preferably is part of the limb connection system. In case the orthotic system comprises a ratchet system and the cuff comprises the retainer, the second part of the ratchet system is preferably located at the retainer, while the first part of the ratchet system is preferably located at the insertion element. Analogous applies to the case where the cuff comprises the insertion element and the device comprises the retainer. The at least one form element at the insertion element and the at least one form element at the retainer may be configured such that they can be interlocked in an engaged state in a plane of engagement. The retainer may form an enclosure or cavity for the insertion element. The retainer may have an insertion slot for insertion of the insertion element. In one embodiment, the retainer has the insertion slot at one of its face sides, through which the insertion element can be inserted into the enclosure or cavity. The slide-in direction may be parallel to the longitudinal extension of the insertion element. The retainer may have a tube-like structure with a rectangular cross-section. In another embodiment, the retainer has the insertion slot at a longitudinal side of the retainer, through which the insertion element can be inserted into the enclosure or cavity. The slide-in direction may be parallel to the transversal extension of the insertion element. The retainer may have a tray-like structure with a rectangular cross-section.

In a preferred embodiment, the insertion element is configured to bring the first part and second part of the ratchet system into the positive locking, in which the first part and second part are engaged, wherein a locking force is realized by an elastic behaviour of the insertion element, and/or wherein the insertion element is configured to be manually pushed and thus moved by a pressing force, so that the first part disengages from the second part or vice versa in order to release the positive locking.

The locking force may be realized by an elastic behaviour of the insertion element. The Young's modulus of the insertion element preferably is between 0.5 GPa and 7 GPa, in case the insertion element is made from a polymer. In case the insertion element is made of a metal or a composite, the Young's modulus of the insertion element may be between 50 GPa and 500 GPa. The insertion element may be configured to push the first part or second part of the ratchet system in the direction of the respective other one of the first part or second part in order to establish the positive locking. At least one component of the locking force may be perpendicular to the slide-in direction and/or the longitudinal extension of the insertion element. The insertion element may be bent while it is inserted into the retainer, wherein the retainer may be configured to absorb the induced bending moment, enabling the application of the locking force.

The insertion element may comprise a bridging section. Support elements may be located at both ends of the bridging section for supporting the insertion element on support parts provided at the retainer. The support elements may protrude at an angle in relation the bridging section, wherein the protruding angle of at least one of the support elements can be in a range of 20 to 160 degrees. Furthermore, the support elements can be arranged in such a way, that the insertion element has a portal-shaped form. The bridging section and/or the support elements may be made from a material having a Young's modulus between 0.5 GPa and 7 GPa. When a force (locking force or pressing force) is applied to such a bridging section, the bridging section and/or said support element are preferably able to elastically deform, either resulting in a disengagement of the first and second part or in an engagement of the first and second part.

The pressing force or at least a component of the pressing force is preferably directed opposite the locking force. The pressing force preferably amounts to at least 3 N. The insertion element may be manually bent and thus partially be moved by applying the pressing force, thereby disengaging the first part and second part of the ratchet system from the positive locking. The device can comprise a button designated for applying the pressing force. Such a button may be provided in the area of the insertion element, where the bridging section is located.

The invention is illustrated by the following examples and figures, from which further embodiments and advantages can be drawn. These examples are meant to illustrate the invention but not to limit its scope.

It is shown in
Fig. 1: a schematic and perspective view of a first embodiment of the device with a tool positioned in a holding mount of the device,
Fig. 2: a schematic and perspective view of a second embodiment of the device,
Fig. 3: a further schematic and perspective view of the device shown in Fig. 2,
Fig. 4: a schematic frontal view of the device shown in Fig. 2 and Fig. 3,
Fig. 5: a schematic side view of the device shown in Fig. 2 through Fig. 4,
Fig. 6: a schematic top view of the device shown in Fig. 2 through Fig. 5,
Fig. 7: a schematic and perspective view of a third embodiment of the device,
Fig. 8: a further schematic and perspective view of the device shown in Fig. 7,
Fig. 9: a schematic and perspective view of the device shown in Fig. 7 and Fig. 8 together with a tool,
Fig. 10: a schematic frontal view of the device shown in Fig. 7 through Fig. 9 together with a tool,
Fig. 11: a further schematic and perspective view of the device shown in Fig. 7 through Fig. 10 together with a tool,
Fig. 12: a schematic and perspective view of a first embodiment of an orthotic system with a cuff and a device,
Fig. 13: a schematic cross-sectional and detailed view of a first embodiment of the cuff,
Fig. 14: a schematic cross-sectional and detailed view of a second embodiment of the cuff,
Fig. 15: a schematic and perspective view of a third embodiment of the cuff,
Fig. 16: a schematic bottom view of a fourth embodiment of the cuff,
Fig. 17a-c: schematic and perspective views of a first embodiment of a retainer and an insertion element having a ratchet system, each Fig. showing a different state,
Fig. 18: a schematic cross-sectional view of a second embodiment of the retainer and the insertion element, and
Fig. 19: a schematic and perspective view of a further embodiment of the device, wherein a socket is part of the limb connection system.

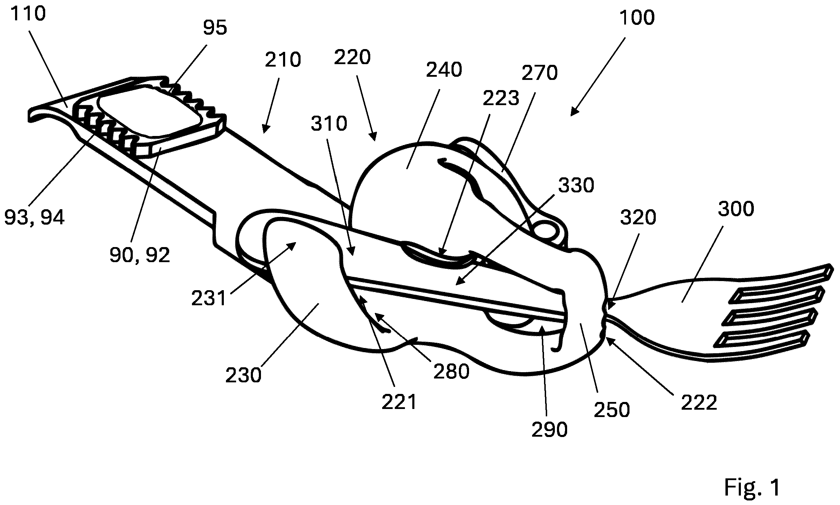

Fig. 1 shows a schematic and perspective view of a first embodiment of the device 100 with a tool 300 positioned in a holding mount 220 of the device 100.

The device 100 comprises a limb connection system 210, which has an insertion element 110 and a first part 92 of a ratchet system 90 arranged at the insertion element 110. The first part 92 of the ratchet system 90 may be integral part of the insertion element 110. The first part 92 comprises multiple form elements 93. In the shown embodiment, each form element 93 has a shape of a tooth 94. The form elements 93 of the first part 92 may be complementary in form and size to a second part 91 of the ratchet system 90. Further, a button 95 is located at the insertion element 110, wherein the button 95 is arranged at the first part 92 of the ratchet system 90.

The holding mount 220 has three bearing portions 221, 222, 223, which are configured to collectively bear the tool 300. In the shown embodiment, the tool 300 is a fork with a flat shape. A first bearing portion 221, which is configured to bear a first section 310 of the tool 300, is located at a first holding member 230. The first holding member 230 is protruding from a main body 240 of the holding mount 220 and has a J-shaped cross-section, so that a distal end 231 of the first holding member 230 is arranged adjacent to the main body 240. A first receptacle 280 for the first section 310 of the tool 300 is formed by the first holding member 230 together with the main body 240. The first holding member 230 has elastic properties such that it is deformable in relation to the main body 240 for allowing placement of the first section 310 in the first receptacle 280.

A second bearing portion 222, which is configured to bear a second section 320 of the tool 300, is located at a second holding member 250. The second holding member 250 is protruding from the main body 240 and has a bridge-shaped cross-section. The second holding member 250 and the main body 240 together form a second receptacle 290. The second holding member 250 has elastic properties such that it is deformable in relation to the main body 240 for allowing placement of the second section 320 in the second receptacle 290 and for allowing placement of the first section 310 in the first receptacle 280. Due to the design and especially the flexibility of the first holding member 230 and the second holding member 250, tools 300 of other types than the fork may be used with the very same holding mount 220. A third bearing portion 223, which is configured to bear a third section 330 of the tool 300, is located at the main body 240.

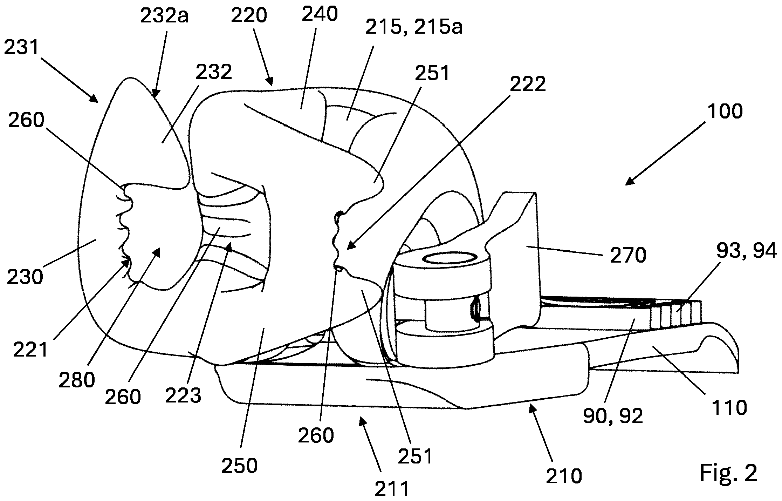

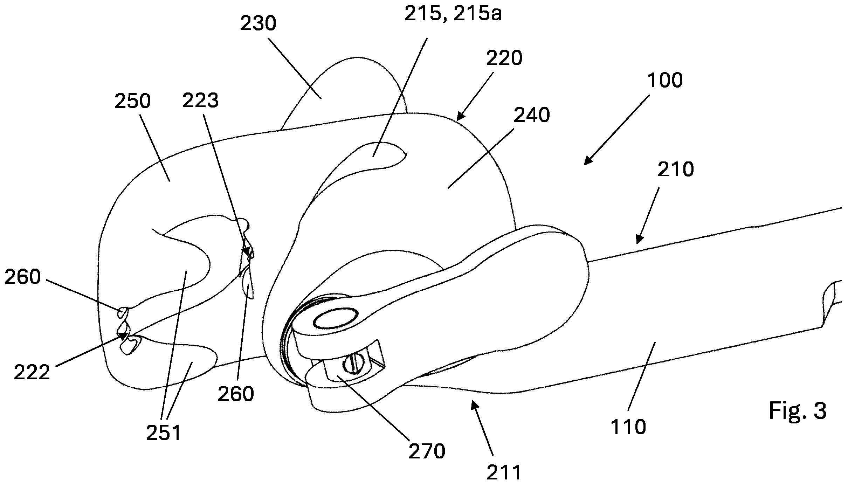

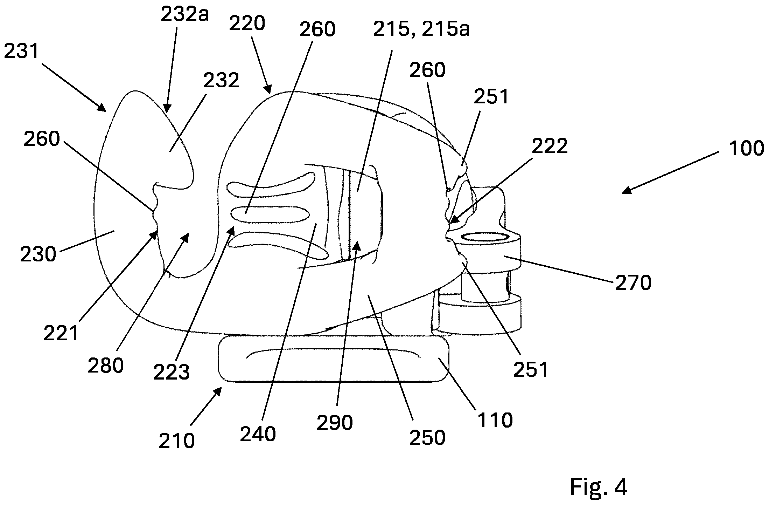

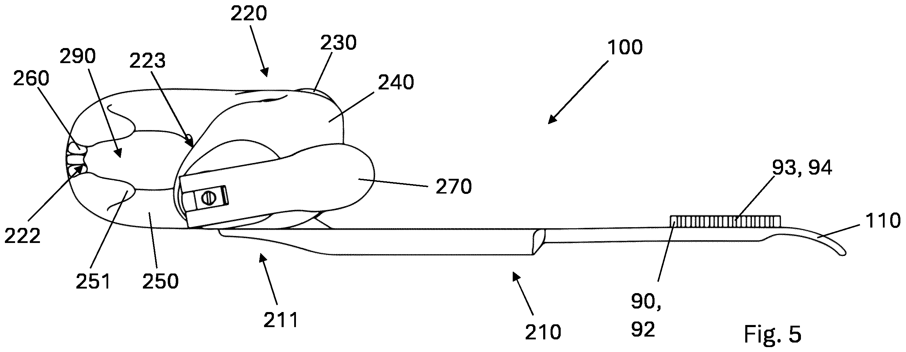

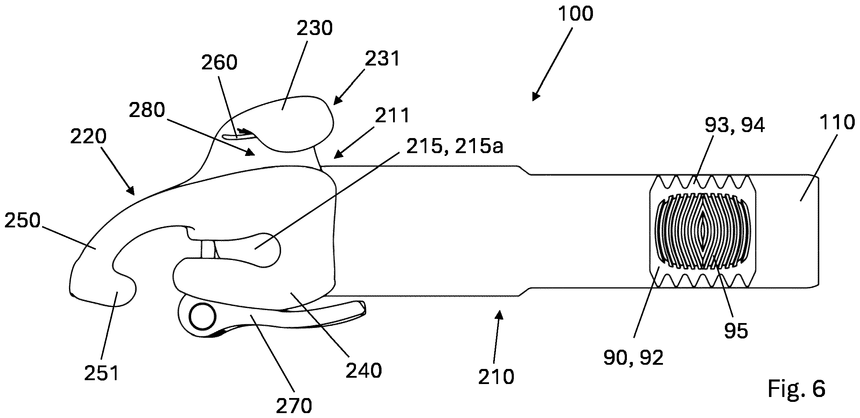

Fig. 2 through Fig. 6 show different schematic views of a second embodiment of the device 100. Fig. 2 and Fig. 3 depict schematic and perspective views, while Fig. 4 depicts a schematic frontal view, Fig. 5 a schematic side view, and Fig. 6 a schematic top view.

The holding mount 220 comprises a main body 240 as well as a first holding member 230 and a second holding member 250, each protruding from the main body 240. The first holding member 230 and the second holding member 250 are protruding in two different planes. The first holding member 230 has a J-shaped cross-section and comprises a nose 232 at its distal end 231, which is arranged adjacent to the main body 240. The nose 232 has a shape that allows smooth insertion of the tool 300 into the first receptacle 280. The second holding member 250 has a bridge-shaped cross-section in one plane and an arc-shaped cross-section in another plane, wherein the planes are arranged essentially orthogonal to each other. The second holding member 250 comprises two teeth 251, which extend substantially orthogonally to the bridge-shaped cross-section and in the same direction. The two teeth 251 are arranged with a distance to each other, so that the second section 320 of the tool 300 may be placed between them. With such teeth 251 the second holding member 250 may have an appearance of an upper jaw of a snake.

The first holding member 230, the second holding member 250 and the main body 240 each comprises three grooves 260, which are located at the first bearing portion 221, the second bearing portion 222, and the third bearing portion 223, respectively. Such grooves 260 allow the use of tools with a flat shape, such as the fork shown in Fig. 1. A flat tool 300 may be snaped from one groove 260 to another groove 260, in order to change its orientation, wherein the change of orientation may be a rotation about a longitudinal axis of the tool 300. At the first holding member 230 the three grooves 260 are located underneath the nose 232. At the second holding member 250 the three grooves 260 are located between the two teeth 251.

The limb connection system 210 and the holding mount 220 are connected to each other via a ball joint 215, by means of which the holding mount 220 is rotatable in relation to the limb connection system 210. The ball joint 215 allows adjustment of a position or an orientation of the holding mount 220 with respect to the limb connection system 210. The ball joint 215 is arranged at a distal end 211 of the limb connection system 210. A ball 215a of the ball joint 215 is connected to or an integral part of the limb connection system 210, in particular the insertion element 110. A counterpart to the ball 215a, which is at least partly covering the ball 215a, is integral part of the holding mount 220, in particular the main body 240.

The device 100 further comprises a locking mechanism 270, which can be used to lock the position of the holding mount 220 with respect to the limb connection system 210. The locking mechanism 270 is configured to brace the counterpart around the ball 215a in order to cause friction between the ball 215a and the counterpart. The locking mechanism 270 comprises a lever to loosen or tighten the locking mechanism 270. When the locking mechanism 270 is in an unlocked status, the holding mount 220 is rotatable in relation to the limb connection system 210. When the locking mechanism 270 is in a locked status, a position or orientation of the holding mount 220 in relation to the limb connection system 210 is locked.

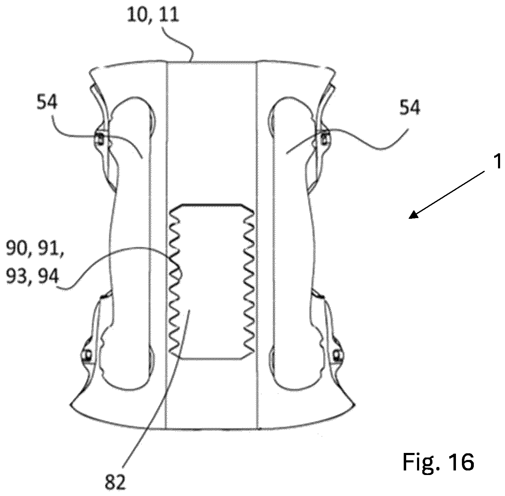

The limb connection system 210 comprises an insertion element 110, which has a transversal extension and a longitudinal extension longer than the transversal extension. The limb connection system 210 has a first part 92 of a ratchet system 90, wherein a second part 91 of the ratchet system 90 may be provided at a cuff 1 (see for example Fig. 16). The first part 92 comprises multiple form elements 93 arranged in a row. Each form element 93 has a shape of a tooth 94. However, other shapes of the form elements 93 are possible. The form elements 93 of the first part 92 may be complementary in form and size to the form elements 93 of the second part 91 or vice versa. Further, a button 95 is located at the insertion element 110, wherein the button 95 is arranged at the first part 92 of the ratchet system 90.

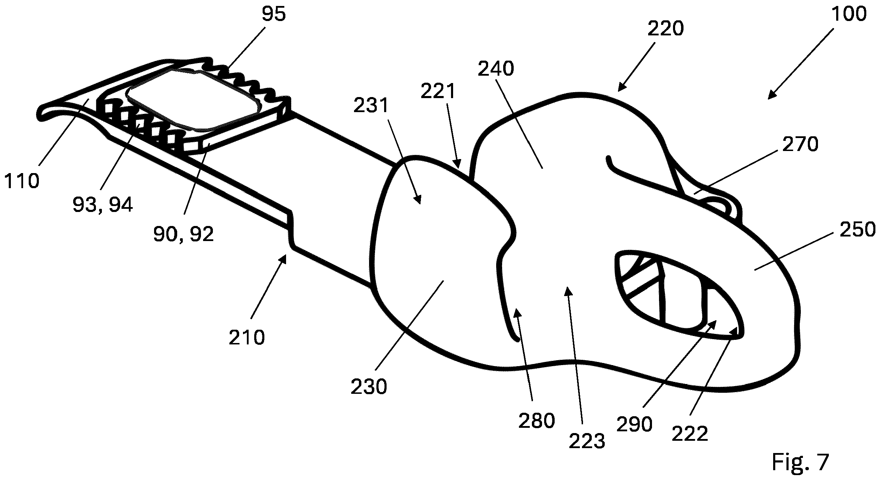

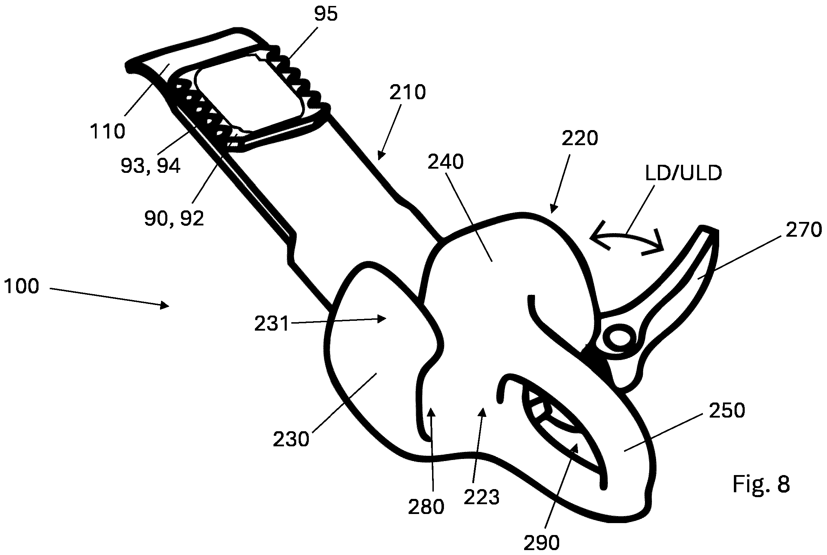

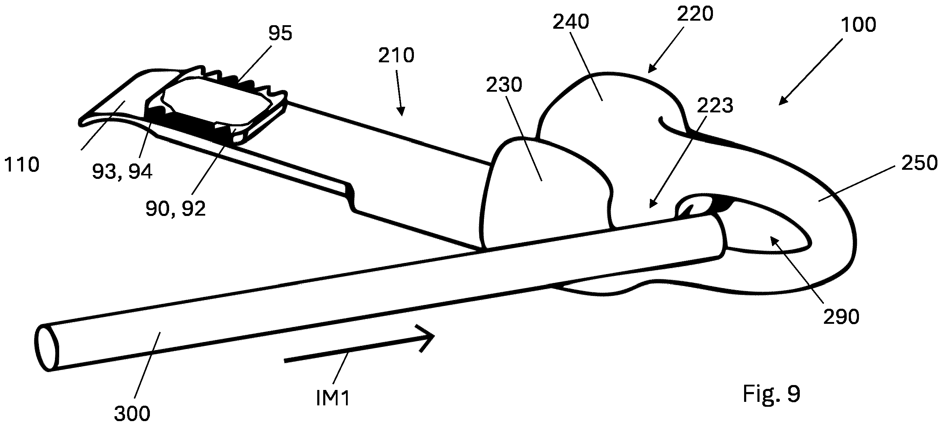

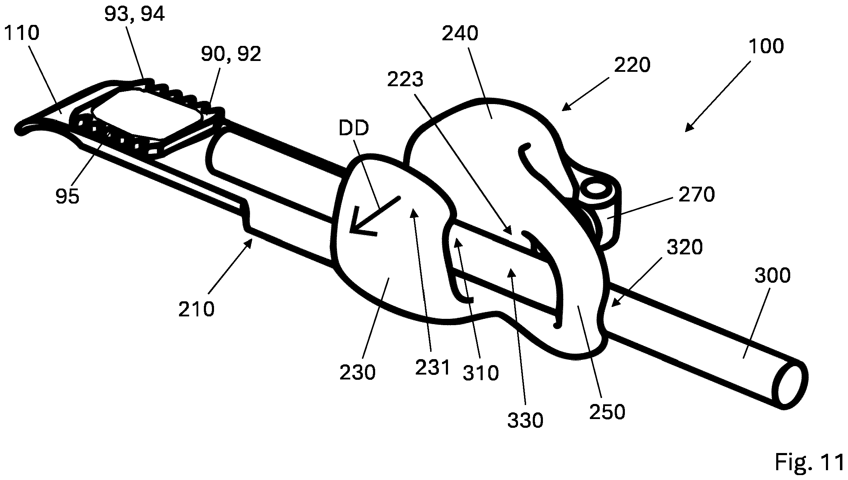

Fig. 7 trough Fig. 11 show different schematic views of a third embodiment of the device 100. While Fig. 7 and Fig. 8 depict schematic and perspective views solely of the device 100, Fig. 9 through Fig. 11 show the device 100 together with the tool 300, which in the shown embodiment is stick, such as a drumstick.

In comparison to the second embodiment shown in Fig. 2 through Fig. 6, the device 100 according to the third embodiment does not comprise a groove 260 or a nose 232 or a tooth 251. Moreover, the second holding member 250 only has a bridge-shaped cross-section. The further description regarding the second embodiment is also valid for the third embodiment. The third embodiment of the device 100 is even more cost-efficient than the second embodiment.

As shown in Fig. 8, the locking mechanism 270 has a lever, which may be swivelled with respect to the main body 240 in locking direction LD and an unlocking direction ULD to bring the locking mechanism 270 from the locked status into the unlocked status and vice versa.

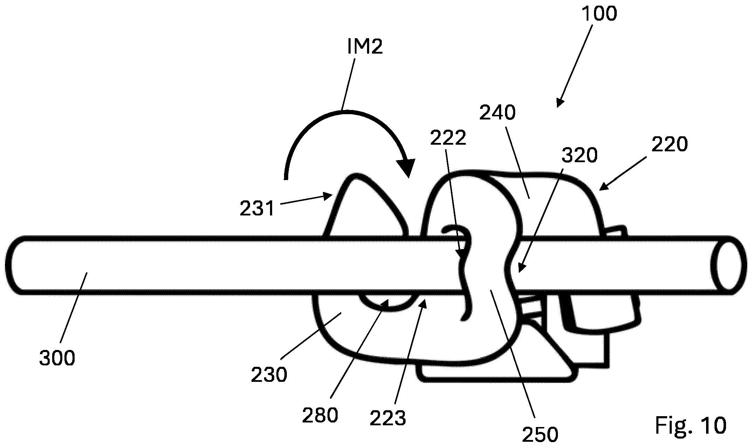

As shown in Fig. 9, the tool 300 can be positioned in the holding mount 220 by inserting the tool 300 into the second receptacle 290 via a first insertion movement IM1. The tool 300 may alternatively inserted from the other side. Via a second insertion movement IM2, the tool 300 may then be inserted into the first receptacle 280, as shown in Fig. 10. This requires deformation of the second holding member 250 as well as of the first holding member 230. The first holding member 230 deforms in the deformation direction DD shown in Fig. 11. In order to remove the tool from the holding mount 220, the described movements IM1, IM2 may be carried out in reverse order.

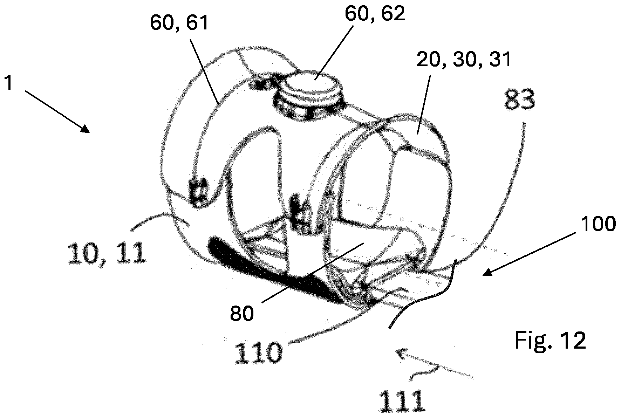

Fig. 12 depicts a schematic and perspective view of a first embodiment of an orthotic system with a cuff 1 and a device 100. The cuff 1 is attachable to a limb, particularly a forearm, of a person and comprises a cuff base 10 and a fixation means 20. The cuff base 10 and the fixation means 20 are combined into a sleeve attachable to the limb, wherein the sleeve can be tightened around the limb by reducing a circumference formed by the cuff base 10 and the fixation means 20.

In the shown embodiment the cuff base 10 is formed by a lower shell 11. Cushioning material 80 is applied in the lower shell 11. The fixation means 20 comprises a cuff top 30 formed by an upper shell 31 as well as a lacing system 60. The lacing system 60 has a lacing element 61, which may be in the form of a cable, and a lacing knob 62, wherein the lacing element 61 is connected to the lacing knob 62. By turning the lacing knob 62, tension is generated in the lacing element 61, which causes the tightening of the lacing system 60. As the tension generated in the lacing system 60 increases, the circumference formed by the cuff base 10 and the fixation means 20 is reduced.

The device 100 is only partly illustrated. As shown, the insertion element 110 of the device 100 is arranged in a retainer 82 provided at the cuff base 10. The insertion element 110 was inserted into the retainer 82 through an insertion slot 83 and along a slide-in direction 111. The device 100 can be removed from the cuff 1 by pulling it out of the retainer 82 in a direction opposite the slide-in direction 111. Due to such a modularity of the orthotic system, it allows for the connection of different devices to best meet the needs of the users. Especially, it can be connected to the described device 100.

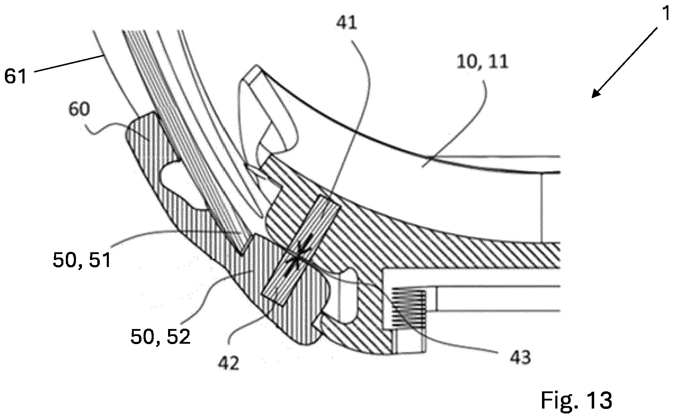

Fig. 13 depicts a schematic cross-sectional and detailed view of a first embodiment of the cuff 1. The cuff 1 has a cuff base 10 in form of a lower shell 11 and a lacing system 60. The cuff base 10 comprises a first form fit means 51, while the lacing system 60 comprises a second form fit means 52, which may act as a hook. The form fit means 51, 52 are adapted to allow a geometrical interlocking between each other, so that the second form fit means 52 and the first form fit means 51 create a form fit connection 50. The second form fit means 52 can be disconnected from the first form fit means 51 whenever it is desired. The second form fit means 52 is provided at a lacing element 61, which may be in the form of a cable.

A first magnetic part 41 is positioned in the cuff base 10. A second magnetic part 42 is positioned in the second form fit means 52. The second form fit means 52 is guided by the attracting magnetic force 43, which is acting between the second magnetic part 42 and the first magnetic part 41 into the first form fit means 51. The magnetic parts 41, 42 then hold the form fit means 51, 52 in place by means of the attracting magnetic force 43, therefore creating a magnetic connection. Once the second form fit means 52 is in place, the lacing element 61 can be tensioned. Once tension is set in the lacing element 61, the form fit means 51, 52 are geometrically interlocked, such that the magnetic connection plays a subordinate role. The higher the tension, the more difficult it becomes to remove the second form fit means 52 from the first form fit means 51. In order to remove the cuff 1, the tension of the lacing system 60 has to be released, so that the second form fit means 52 can be disconnected from the first form fit means 51.

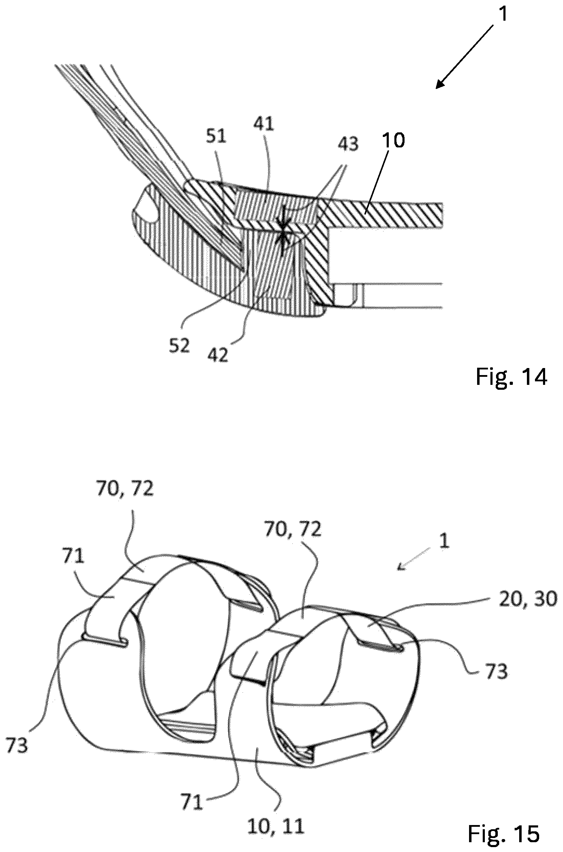

Fig. 14 shows a schematic cross-sectional and detailed view of a second embodiment of the cuff 1. In comparison to the first embodiment shown in Fig. 13, the cuff 1 according to the second embodiment has form fit means 51, 52 of different shape, which are positioned at a different position with respect to the cuff base 10. Also, the magnetic parts 41, 42 are arranged differently.

Fig. 15 depicts a schematic and perspective view of a third embodiment of the cuff 1. In this embodiment, the cuff 1 has a cuff base 10 in form of a lower shell 11 and a fixation means 20 in form of a strap system 70. The strap system 70 comprises two straps 71 which are led through cutouts 73 of the cuff base 10. The straps 71 are provided with a hook-and-loop system 72, respectively. By tensioning the straps 71 and fixation of the hook-and-loop system 72, the cuff 1 can be tightened around the forearm.

Fig. 16 shows a schematic bottom view of a fourth embodiment of the cuff 1. The cuff 1 comprises the second part 91 of a ratchet system 90, which is compatible to establish a mechanical connection to the first part 92 of the ratchet system 92 arranged at the device 100. The second part 91 of the ratchet system 90 is provided at the lower shell 11 of the cuff 1. The second part 91 has multiple form elements 93 in the shape of teeth 94. The form elements 93 are arranged in a row.

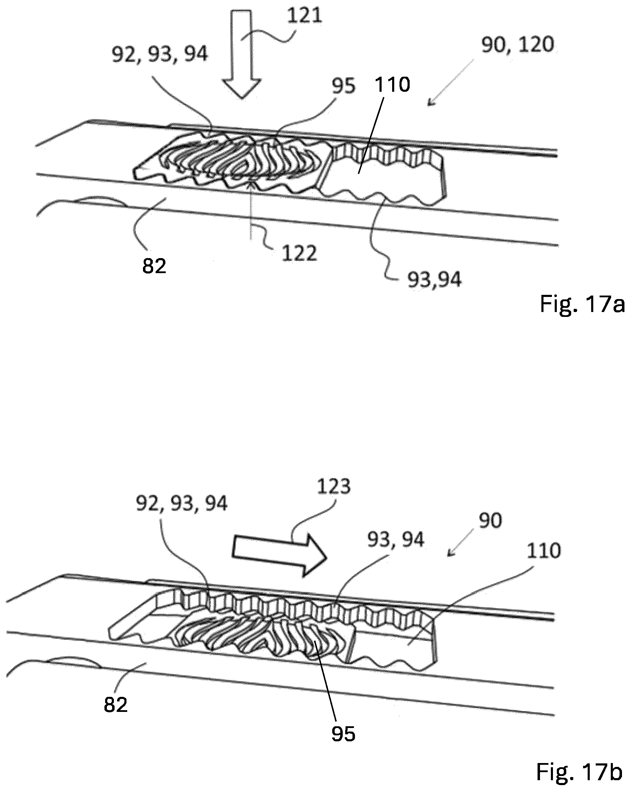

Fig. 17a-c show schematic and perspective views of a first embodiment of a retainer 82 and an insertion element 110 having a ratchet system 90, each Fig. showing a different state. These figures show a method for changing the position of the insertion element 110 with respect to the retainer 82. Depending on the activity and intensity, different positions of the device 100 might be required. Also, adjustment of the position may allow users with different arm lengths to use the same device 100.

The insertion element 110 and the retainer 82 are not shown completely. At the insertion element 110 the first part 92 of the ratchet system 90 is provided, comprising also a plurality of form elements 93 in the shape of teeth 94, respectively. At the retainer 82 the second part 91 of the ratchet system 90 is provided, comprising a plurality of form elements 93 in the shape of teeth 94, respectively. The number of positions of the insertion element 110 with respect to the retainer 82 is determined by the number of form elements 93 on the first part 92 and the second part 91 of the ratchet system 90. The form elements 93 of the first part 92 and of the second part 91 of the ratchet system 90 are arranged in rows, respectively, and are complementary to each other in form and size. The insertion element 110 comprises the button 95, which is located at the first part 92 of the ratchet system 90.

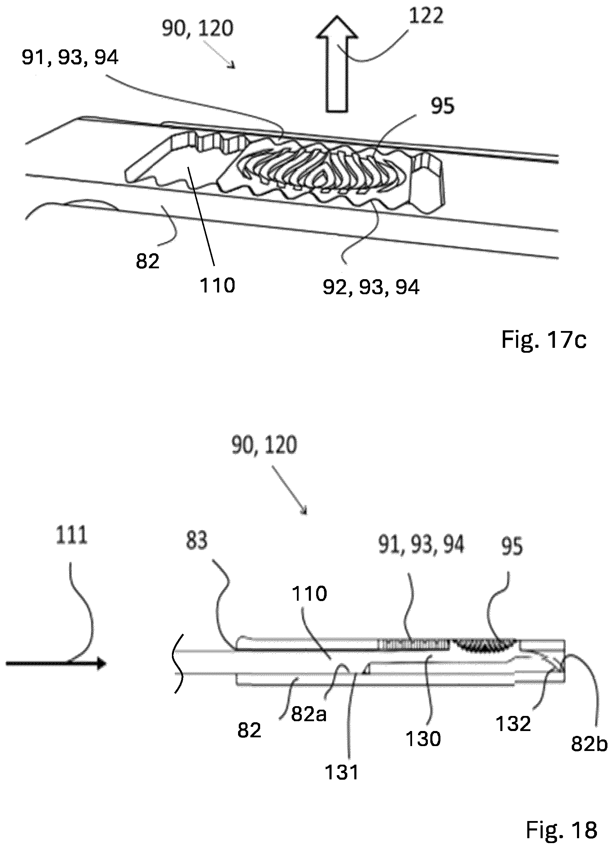

Fig. 17a shows a state in which the form elements 93 of the first part 92 and the second part 91 engage with each other, so that a positive locking 120 is established. This engaged state is maintained by means of a locking force 122, which may act due to elasticity of the insertion element 110 (compare Fig. 18). When the button 95 is, for instance manually, pressed by a pressing force 121 acting against the locking force 122, the button 95 and therefore the insertion element 110 is pressed inward, so that the form elements 93 of the first part 92 and of the second part 91 of the ratchet system 90 are disengaged. Consequently, the insertion element 110 can be moved parallel to a longitudinal extension of the insertion element 110, by applying a moving force 123 via the button 95. Thus, the position of the insertion element 110 with respect to retainer 82 can be adjusted. Also, the insertion element 110 may be removed from the retainer 82. When the foreseen position has been reached, the button 95 can be released and due to locking force 122 the insertion element 110 as well as the button 95 moves, such that the form elements 93 of the first part 92 and of the second part 91 of the ratchet system 90 engage again, shown in fig. 17c.

Fig. 18 depicts a schematic cross-sectional view of a second embodiment of the retainer 82 and the insertion element 110. The insertion element 110 was inserted into the retainer 82 through an insertion opening 83 along the slide-in direction 111. In the illustrated state, the insertion element 110 is completely inserted into the retainer 82. The retainer 82 encloses the insertion element 110, such that at least one rotational degree of freedom of the inserted insertion element 110 around an axis perpendicular to the slide-in direction 111 is blocked. Moreover, the insertion element 110 has a portal-shaped form with a bridging section 130 and a first support element 131 located at one end of the bridging section 130 and a second support element 132 located at another end of the bridging section 130. The support elements 131, 132 support the insertion element 110 on two support parts 82a, 82b, wherein the support parts 82a, 82b are integral parts of the retainer 82.

A ratchet system 90 is provided, wherein the first part 92 of the ratchet system 90 is provided at the insertion element 110, and the second part 91 of the ratchet system 90 is provided at the retainer. The form elements 93 of the first part 92 and the second part 91 are in the shape of teeth 94. The first part 92 and the second part 91 are depicted in the positive locking 120. The positive locking 120 of the form elements 93 allows the transfer of loads along the slide-in direction 111. The button 95 is located on top of the bridging section 130 of the insertion element 110. When a person's finger presses the button 95, the bridging section 130 and the second support element 132 undergo an elastic deformation. As a result, the first and second part 91, 92 disengage from the positive locking 120 and the insertion element 110 can be moved along the slide-in direction 111 as long as the button 95 remains to be pressed.

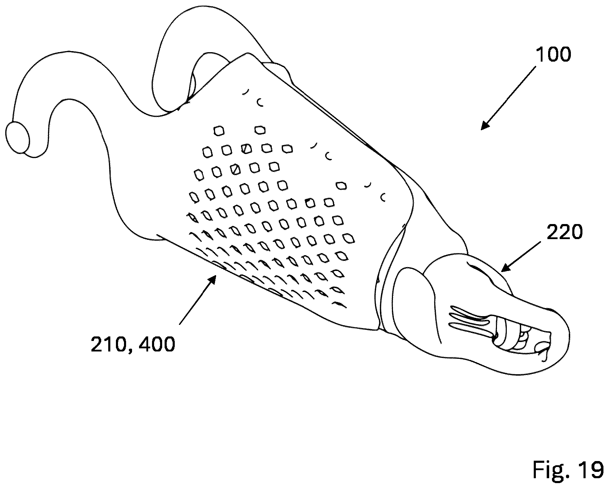

Fig. 19 shows a schematic and perspective view of a further embodiment of the device 100, wherein a socket 400 is part of the limb connection system 210. The socket 400 may be worn at a limb of a person, in particular a forearm. The holding mount 220 is mechanically connected to the socket 400. A ball joint 215 (see for example Fig. 6) may be used to connect the socket 400 to the holding mount 220, in order to allow adjustment of the position or orientation of the holding mount 220 with respect to the socket 400.

### List of reference signs

- 1: cuff
- 10: cuff base
- 11: lower shell
- 20: fixation means
- 30: cuff top
- 31: upper shell
- 41: first magnetic part
- 42: second magnetic part
- 43: attracting magnetic force
- 50: form fit connection
- 51: first form fit means
- 52: second form fit means
- 60: lacing system
- 61: lacing element
- 62: lacing knob
- 70: strap system
- 71: strap
- 72: hook-and-loop system
- 73: cutout
- 80: cushioning material
- 82: retainer
- 82a: first support part
- 82b: second support part
- 83: insertion slot
- 90: ratchet system
- 91: second part (ratchet system)
- 92: first part (ratchet system)
- 93: form element
- 94: tooth
- 95: button
- 100: device
- 110: insertion element
- 111: slide-in direction
- 120: positive locking
- 121: pressing force
- 122: locking force
- 123: moving force
- 130: bridging section
- 131: first support element
- 132: second support element
- 210: limb connection system
- 211: distal end (limb connection system)
- 215: ball joint
- 215a: ball (ball joint)
- 220: holding mount
- 221: first bearing portion
- 222: second bearing portion
- 223: third bearing portion
- 230: first holding member
- 231: distal end (first holding member)
- 232: nose
- 240: main body
- 250: second holding member
- 251: tooth
- 260: groove
- 270: locking mechanism
- 280: first receptacle
- 290: second receptacle
- 300: tool
- 310: first section (tool)
- 320: second section (tool)
- 330: third section (tool)
- 400: socket
- DD: deformation direction
- IM1: first insertion movement
- IM2: second insertion movement
- LD: Locking direction
- ULD: unlocking direction

## Claims

1. Device (100) for establishing a mechanical connection between a limb of a person and a tool (300), such that a load transfer is enabled from the limb to the tool (300) and vice versa, the device comprising a limb connection system (210) configured to achieve a form-fitting and/or force-fitting attachment to the limb, further comprising a holding mount (220) in which the tool (300) can be positioned, wherein the holding mount (220) comprises at least three bearing portions (221, 222, 223), which are configured to collectively bear the tool (300), wherein a first bearing portion (221) is located at a first holding member (230) protruding from a main body (240) of the holding mount (220), wherein the first holding member (230) and the main body (240) together form a first receptacle (280) for a first section (310) of the tool (300), and wherein the first holding member (230) has elastic properties such that it is deformable in relation to the main body (240) for allowing placement of the first section (310) in the first receptacle (280).

2. Device (100) according to claim 1, wherein a distal end (231) of the first holding member (230) is arranged adjacent to the main body (240).

3. Device (100) according to any one of claims 1 and 2, wherein a second bearing portion (222) is located at a second holding member (250) protruding from the main body (240), wherein the second holding member (250) and the main body (240) together form a second receptacle (290) for a second section (320) of the tool (300).

4. Device (100) according to any one of the preceding claims, wherein the first holding member (230) and/or the second holding member (250) has a bridge-shaped and/or arc-shaped and/or J-shaped and/or S-shaped cross-section.

5. Device (100) according to any one of claims 3 and 4, wherein the second holding member (250) has elastic properties such that it is deformable in relation to the main body (240) for allowing placement of the second section (320) in the second receptacle (290) and/or for allowing placement of the first section (320) in the first receptacle (280).

6. Device (100) according to any one of the preceding claims, wherein a third bearing portion (223) is located at the main body (240).

7. Device (100) according to any one of the preceding claims, wherein the second holding member (250) protrudes from the main body (240) in a different plane than the first holding member (230) and/or wherein at least one of the first holding member (230) and second holding member (250) comprises at least one nose (232) and/or lip and/or tooth (251), which at least supports to bear the tool (300) such that the tool (300) is exposable to external forces in at least two directions, which may be essentially oriented orthogonally to each other.

8. Device (100) according to any one of the preceding claims, wherein at least one of the at least three bearing portions (221, 222, 223) has a groove (260), which enables fixation of a tool (300) with a flat shape.

9. Device (100) according to any one of the preceding claims, comprising a ball joint (215), by means of which the holding mount (220) is rotatable in relation to the limb connection system (210).

10. Device (100) according to any one of the preceding claims, wherein the holding mount (220) is made one-piece and/or the holding mount (220) or parts of the holding mount (220) are made from a rubber-like material.

11. Orthotic system, comprising a device (100) according to one of the preceding claims and a cuff (1) mechanically connectable or connected to the device (100) by means of the limb connection system (210).

12. Orthotic system according to claim 12, wherein the cuff (1) comprises a cuff base (10) and a fixation means (20), wherein the cuff base (10) and the fixation means (20) can be combined or are combined via direct or indirect interlocking with each other into a sleeve attachable to the limb, wherein the sleeve can be tightened around the limb by reducing a circumference formed by the cuff base (10) and the fixation means (20), such that the form-fitting and/or force-fitting attachment to the limb is achieved.

13. Orthotic system according to any one of claims 12 and 13, wherein the device (100) comprises a first part (92) of a ratchet system (90) and the cuff (1) comprises a second part (91) of the ratchet system (90), wherein the first part (92) and the second part (91) each have at least one form element (93), which are complementary in form and size, in order to establish a positive locking (120).

14. Orthotic system according to any one of claims 12 through 14, wherein the cuff (1) or the device (100) comprises a retainer (82) and the respective other one of the cuff (1) and the device (100) comprises an insertion element (110), wherein the insertion element (110) is configured to be pushed into the retainer (82) along a slide-in direction (111).

15. Orthotic system according to claim 15, wherein the insertion element (110) is configured to bring a first part (92) and second part (91) of a ratchet system (90) into a positive locking (120), in which the first part (92) and second part (91) are engaged, wherein a locking force (122) is realized by an elastic behaviour of the insertion element (110), and/or wherein the insertion element (110) is configured to be manually pushed and thus moved by a pressing force (121), so that the first part (92) disengages from the second part (91) or vice versa in order to release the positive locking (120).
